# EUROPÄISCHE PATENTSCHRIFT

(11) **EP 0 945 121 B1**
(45) Veröffentlichungstag und Bekanntmachung des Hinweises auf die Patenterteilung: **03.01.2007**
(21) Anmeldenummer: 99101735.1
(22) Anmeldetag: 11.02.1999
(51) Int. Cl.: A61K 8/19, A61K 8/365, A61K 8/63, A61K 8/67, A61Q 5/06, A61Q 5/12

(54) **Stylingmittel auf Wasserbasis enthaltend Gamma-Oryzanol und Calciumsalzen**
Aqueous hairstyling composition containing gamma-oryzanol and calcium salts
Composition de mise en forme des cheveux contenant du gamma-oryzanol et des sels de calcium

(30) Priorität: 11.02.1998 DE 19805428
(43) Veröffentlichungstag der Anmeldung: 29.09.1999
(73) Patentinhaber: Beiersdorf Aktiengesellschaft, 20245 Hamburg (DE)
(72) Erfinder: Riedel, Jan-Henric, Dr., 21075 Hamburg (DE); Kollar, Andreas, Dr., 21035 Hamburg (DE); Argembeaux, Horst, 22525 Hamburg (DE)

(56) Entgegenhaltungen:
- DE-A- 1 617 557
- FR-A- 1 145 887
- PATENT ABSTRACTS OF JAPAN vol. 4, no. 42 (C-5) [524] & JP 55 017365 A (HOTSUKOU YUSHI KK)
- PATENT ABSTRACTS OF JAPAN vol. 018, no. 118 (C-1172) & JP 05 310526 A (EISAI CO)
- "The Merck Index, Twelfth edition" 1996, MERCK RESEARCH LABORATORIES , WHITEHOUSE STATION, NJ * Absatz [7016] *

## Beschreibung

### Die vorliegende Erfindung betrifft

Stylingzubereitungen oder Stylingmittel mit einem Gehalt an Substanzen, die neben der Festigungswirkung für die Haare die Kopfhaut und/oder das Haar, aber auch die Zubereitungen selbst vor unerwünschten Oxidationsprozessen schützen. In einer bevorzugten Ausführungsform betrifft die vorliegende Erfindung Wirkstoffkombinationen und Zubereitungen damit, die dazu dienen, neben der Festigungswirkung für die Haare das Haar und die Kopfhaut zu pflegen.

Oxidative Prozesse schädigen Stoffe unterschiedlichster Natur (z.B. Haut, Haare und Wolle, aber auch Lacke und Kunststoffe, um nur einige zu nennen) zum Teil in erheblichem Maße. Die Stoffe ändern dabei ihre physikalischen und chemischen Eigenschaften: sie "altern". Zur Verzögerung oder sogar Inhibierung der Alterung von Stoffen werden im allgemeinen sogenannte Alterungsschutzmittel verwendet.

Insbesondere die schädigende Wirkung des ultravioletten Teils der Sonnenstrahlung auf organische, aber auch anorganische Stoffe ist allgemein bekannt. Für den Menschen sind dabei die Schädigung von Haut und Haaren und die Verzögerung oder Verhinderung derselben durch den Einsatz von Lichtschutzmitteln von besonderer Bedeutung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluß des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluß auf das Haar, der sich beispielsweise darin äußert, daß bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Gewöhnlich werden dabei Wasserstoffperoxidkonzentrationen um 6% verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagiert und dabei unter Umständen eine Schädigung des Haares bewirken kann.

Oxidative Einflüsse, wie beispielsweise chemische Haarbehandlungen oder Sonnenlicht, setzen demnach die Festigkeit und die Elastizität des Haares herab und führen zu einer Zerstörung von Melanin. Augenscheinlich wird dies insbesondere bei dunkelhaarigen Personen, deren Haar im Sommer durch das intensive Sonnenlicht deutlich aufgehellt wird. Unter dem Bergriff "oxidativer Einfluß" ist im Sinne der vorliegenden Erfindung sowohl der Einfluß von oxidierend wirkenden Substanzen zu verstehen als auch die oxidative Wirkung von durch Strahlung, namentlich Licht, insbesondere UV-Licht, hervorgerufenen Folgeprodukten.

Ein Ziel der Haarpflege ist es, Kopfhaut und -haar vor oxidativen Einflüssen zu schützen und den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, daß sie nicht nur der Pflege des einzelnen Haars dienen, sondern auch das Aussehen einer Frisur insgesamt verbessern, beispielsweise dadurch, daß sie dem Haar mehr Fülle verleihen, die Frisur über einen längeren Zeitraum fixieren oder die Frisierbarkeit verbessern.

Die Verwendung von Antioxidantien, also Substanzen, die Oxidationsprozesse verhindern, in Kosmetika ist an sich bekannt. Antioxidantien, die in der Kosmetik Verwendung finden, sind beispielsweise Tocopherole, Gallensäurederivate, Sesamol und Flavonoide. Antioxidantien werden hauptsächlich als Schutzsubstanzen gegen der Verderb der sie enthaltenden Zubereitungen verwendet.

Auch Tocopherole, insbesondere Vitamin E, sind natürlich prinzipiell geeignet, Oxidationsprozesse zu verhindem, und finden dementsprechend häufig in Kosmetika Verwendung. Allerdings haben Tocopherole den Nachteil, daß sie im allgemeinen sehr reaktiv sind und daher zum Teil bereits in der Zubereitung abreagieren. Dies führt dazu, daß nur ein kleiner Teil der Einsatzmenge den zu schützenden Körperteil überhaupt erreicht, so daß die erzielte Wirkung weit hinter der erhofften zurückbleibt.

Eine Aufgabe der vorliegenden Erfindung war, die Nachteile des Standes der Technik zu beseitigen. Insbesondere sollten kosmetische Wirkstoffe bzw. Zubereitungen, solche Wirkstoffe enthaltend, zur Verfügung gestellt werden, bei deren Verwendung die Schädigung der Kopfhaut und/oder des Haares durch oxidativen Einfluß gemindert, wenn nicht gänzlich verhindert werden kann.

Erfindungsgemäß wird diese Aufgabe gelöst und werden die Nachteile des Standes der Technik beseitigt.

Zwar beschreibt die deutsche Offenlegungsschrift 1617557 eine Nährlösung enthaltend Oryzanol und Calcium-Salze, doch konnte diese Schrift nicht den Weg zur vorliegenden Erfindung weisen, da es sich nicht um ein Styling-Mittel handelt.

Gegenstand der Erfindung sind Stylingmittel auf Wasserbasis, dadurch gekennzeichnet, daß sie Gamma-Oryzanol und ein Calciumsalz oder mehrere Calciumsalze, ausgewählt aus der Gruppe, gebildet von Calciumpanthotenat, Calciumchlorid und Calciumlactat, enthalten.

Gegenstand der Erfindung ist weiterhin die Verwendung von Wirkstoffkombinationen in Stylingmitteln auf Wasserbasis, bestehend aus Gamma-Oryzanol und einem Calciumsalz oder mehreren Calciumsalzen, ausgewählt aus der Gruppe, gebildet von Calciumpanthotenat, Calciumchlorid und Calciumlactat, zum Schutz haarkosmetischer Zubereitungen und/oder der Kopfhaut und/oder des Haares vor unerwünschten Oxidationsprozessen.

Bevorzugte haarkosmetische Zubereitungen sind Stylingmittel bzw. Haarverfestigungsmittel (Haarfestiger) auf Wasserbasis.

Die erfindungsgemäßen Wirkstoffkombinationen und Zubereitungen, diese Wirkstoffkombinationen enthaltend, mindern die Schädigung der Kopfhaut und/oder des Haares durch oxidative Einflüsse besser als Wirkstoffe, Wirkstoffkombinationen und Zubereitungen des Standes der Technik. Insbesondere pflegen sie durch oxidativen Streß geschädigtes oder strapaziertes Haar bzw. beugen solchen Schäden vor.

Gamma-Oryzanol ist in der Literatur beschrieben (CAS-Nr.: 11042-64-1 bzw. 12738-23-7). Gamma-Oryzanol ist keine einheitliche Verbindung, sondern ein Gemisch verschieden strukturierter Ferulasäureester. Gamma-Oryzanol besteht hauptsächlich aus den Estern der Ferulasäure mit den Triterpenalkoholen Cycloartenol und 24-Mathylencylcloartenot. Ferner enthält es geringe Mengen an Estern mit Sterolen, namentlich mit Camesterol, Stigmasterol und β-Sitosterol.

Gamma-Oryzanol ist im Handel erhältlich, beispielsweise unter dem Handelsnamen Gamma-Oryzanol (Lieferant: Jan Dekker oder Henry Lamotte, DE).

Vorteilhaft enthalten erfindungsgemäße Zubereitungen 0,001 bis 5,0 Gew.-% Gamma-Oryzanol, bevorzugt 0,01 bis 2,0 Gew.-%, jeweils bezogen auf das Gesamtgewicht der Zubereitungen.

Vorteilhaft enthalten erfindungsgemäße Zubereitungen 0,01 bis 25 Gew.-% eines oder mehrerer Calciumsalze, bevorzugt 0,02 bis 5 Gew.-%, jeweils bezogen auf das Gesamtgewicht der Zubereitungen.

Als Calciumsalz wird Calciumpanthotenat bevorzugt. Die Calciumsalze sind im Handel erhältlich.

Die Stylingmittel und Zubereitungen, die erfindungsgemäße Wirkstoffkombinationen enthalten, sind topische Zubereitungen. Diese können wie üblich zusammengesetzt sein und zur Behandlung und der Pflege der Kopfhaut und/oder der Haare oder als Lichtschutzpräparat dienen. Zur Anwendung werden die erfindungsgemäßen Zubereitungen in der für Kosmetika und Stylingmittel üblichen Weise auf die Kopfhaut und die Haare in ausreichender Menge aufgebracht.

Vorteilhaft können Zubereitungen im Sinne der vorliegenden Erfindung als Schaumfestiger, Flüssigfestiger, Stylinggele, Schaumaerosole, Emulsionen, Lösungen vorliegen.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältem ausschäumbare oder versprühbare kosmetische oder dermatologische Zubereitungen im Sinne der vorliegenden Erfindung, wie z.B. Schaumfestiger, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhaft enthalten erfindungsgemäße Zubereitungen neben einem wirksamen Gehalt an erfindungsgemäßen Wirkstoffkombinationen ferner übliche Wirk-, Inhalts-, Zusatz- und/oder Hilfsstoffe.

Erfindungsgemäße kosmetische Zubereitungen zur Festigung und zum Styling der Haare enthalten Filmbildner, wie sie normalerweise in solchen Zubereitungen verwendet werden, wobei die Gesamtmenge der Filmbildnersubstanzen z. B. zwischen 0,5 und 20 Gew.-% beträgt, bezogen auf das Gesamtgewicht der Zubereitungen.

Erfindungsgemäß können als günstige Filmbildner alle für kosmetische und/oder dermatologische Anwendungen geeigneten oder gebräuchlichen Filmbildner verwendet werden.

Vorteilhaft werden der oder die Filmbildner gewählt aus der Gruppe der wasserlöslichen oder wasserdispergierbaren Polyurethane, Polyharnstoffe, Silikonharze und/oder Polyester sowie der nichtionischen, anionischen, amphoteren und/oder kationischen Polymere.

Vorteilhafte nichtionische Polymere, die in erfindungsgemäßen Zubereitungen alleine oder im Gemisch, vorzugsweise auch mit anionischen und/oder amphoteren und/oder zwitterionischen Polymeren enthalten sein können, sind Vinylpyrrolidon-Homo- oder Copolymerisate. Hierzu gehören beispielweise Polyvinylpyrrolidon, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat, Polysiloxane und dergleichen mehr.

Vorteilhafte anionische Polymere sind beispielsweise Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere, Natriumpolystyrolsulfonat, Ethylacrylat/N-tert.-Butylacrylamid/Acrylsäure-Copolymere, VinylpyrrolidonlVinylacetat/Itaconsäure-Copolymere, Acrylsäure/Acrylamid-Copolymere und/oder deren Natriumsalze, Homo- und/oder Copolymere von Acrylsäure und/oder Methacrylsäure und/oder deren Salze sowie Acrylat/Hydroxyacrylat-, Octylacrylamid/Acrylat- bzw. Methacrylsäurester und/oder Butylacrylat/N-Vinylpyrrolidon-Copolymere.

Weitere bevorzugte anionische Polymere sind Methylvinylether/Maleinsäure-Copolymere, die durch Hydrolyse von Vinylether/Maleinsäureanhydrid-Copolymeren entstehen. Diese Polymere können auch teilverestert sein (Ethyl, Isopropyl- bzw. Butylester).

Vorteilhafte amphotere Polymere, die in erfindungsgemäßen Zubereitungen alleine oder im Gemisch, vorzugsweise auch mit anionischen und/oder nichtionischen Polymeren enthalten sein können, sind Copolymerisate aus N-Octylacrylamid, (Meth)Acrylsäure und tert.-Butylaminoethylmethacrylat vom Typ "Amphomer", Copolymerisate aus Methacryloylethylbetain und Alkylmethacrylaten vom Typ "Yukaformer", Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie z.B. Mono- bzw. Dialkylaminoalkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure, wobei diese Aufstellung selbstverständlich nicht limitierend sein soll.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren. Hierzu können beispielsweise Alkali- bzw. Erdalkalibasen, Ammoniak und/oder verschiedene Amine, wie z.B. Triethanolamin, Triisopropanolamin, Aminomethyl-propanol und/oder Aminomethylpropandiol, eingesetzt werden. Die Neutralisation kann je nach Anwendungszweck teilweise oder vollständig erfolgen.

Vorteilhafte kationische Polymere sind beispielsweise Vinylpyrrolidon/Vinylimidazoliummethochlorid-Copolymere, quaternisierte Vinylpyrrolidon/Dialkylaminoalkylmethacrylat-Copolymere, kationische Cellulose-Derivate, wie z.B. Hydroxyethylcellulose/Dimethylalkylammoniumchlorid-Copolymere sowie Terpolymere aus VinylcaprolactamlVinylpyrrolidon mit Dimethylaminoethylmethacrylat bzw. Vinylimmidazoliniummethochlorid und Acrylamidocopolymere.

Es ist auch vorteilhaft, Filmbildner auf natürlicher Basis, z.B. Chitosan und dessen Derivate, einzusetzen, insbesondere im Gemisch mit synthetischen Polymeren.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die erfindungsgemäßen wäßrigen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Die Menge der Verdickungsmittel beträgt beispielsweise 0,01 bis 20 Gew.-%, vorzugsweise 0,01 bis 10 Gew.-%, insbesondere 0,05 bis 5 Gew.-%, jeweils bezogen auf das Gesamtgewicht der Zubereitung.

Der Wassergehalt der Zubereitungen beträgt beispielsweise 50 bis 95 Gew.-%, vorzugsweise 60 bis 95 Gew.-%, insbesondere 70 bis 95 Gew.-%, jeweils bezogen auf das Gesamtgewicht der Zubereitung.

Der Anteil der Alkohole in den Zubereitungen beträgt beispielsweise 0 bis 30 Gew.-%, vorzugsweise 0 bis 20, insbesondere 0 bis 15 Gew.-%, jeweils bezogen auf das Gesamtgewicht der Zubereitung.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 2 Gew.-%, vorzugsweise 0,01 bis 1 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Nydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

In den erfindungsgemäßen wäßrigen Stylingformulierungen können als Filmbildner bevorzugt nichtionische Polymere vom Typ PVP/VA Copolymer eingesetzt werden, die z.B. unter dem Handelsnamen Luviskol VA 64W von der Fa. BASF bzw. unter der Bezeichnung PVP/VA W735 von der Fa. International Speciality Products (ISP) verfügbar sind. Dabei kommen z.B. Konzentrationen von 2 - 8 Gew.-% des Gesamtgewichts der Zubereitung (bezogen auf den Aktivgehalt des Polymers) zum Einsatz.

Bevorzugt kommen auch kationische Polymere vom Typ Polyquaternium-11 (Handelsnamen Gafquat 755N, Fa.International Speciality Products (ISP)) oder -16 (Handelsname Luviquat FC 550, Fa. BASF) zum Einsatz, die neben den festigenden auch konditionierende Eigenschaften aufweisen und somit zusätzlich als Pflegestoffe fungieren. Die Konzentrationen bewegen sich dabei z. B. zwischen 1 und 5 Gew.-% der Gesamtformulierung (bezogen auf den Aktivgehalt des Polymers) bei dem alleinigen Einsatz, bzw. z.B. zwischen 0.2 und 2 Gew.-% des Gesamtgewichts der Zubereitung (bezogen auf den Aktivgehalt des Polymers), wenn zusätzlich auch nichtionische Polymere eingesetzt werden.

In den erfindungsgemäßen wässrigen Stylingformulierungen können als Pflegestoffe bevorzugt monomere quaternäre Stickstoffverbindungen (Quats), wie z.B. Cetrimonium Chloride, das von der Fa. Henkel mit der Handelsbezeichnung Dehyquat A angeboten wird, oder Hydroxyethyl Cetyldimonium Phoshate, das unter der Handelsbezeichnung Luviquat MonoCP von der Fa. BASF verfügbar ist, zum Einsatz kommen.

Die Einsatzkonzentrationen liegen dabei z.B. zwischen 0.05 - 1.0 Gew.-% des Gesamtgewichts der Zubereitung (bezogen auf den Aktivgehalt des Quats).

In den erfindungsgemäßen wässrigen Stylingformulierungen, z.B. in Stylinggelen,können statt Verdickern auf Polyacrylsäure-Basis als Alternative zu den Quats auch Silikontenside (Polyethermodifizierte Siloxane) vom Typ Dimethicone Copolyol bevorzugt zum Einsatz kommen. Dimethicone Copolyole werden z.B. unter der Handelsbezeichnung DC 193 von der Fa. Dow Corning bzw. Abil B 8851 von der Fa. Goldschmidt angeboten. Die Einsatzkonzentrationen liegen z.B. zwischen 0.02 und 1.0 Gew.-% der Gesamtformulierung.

Zweckmäßigerweise sind Komplexbildner, insbesondere Komplexierungsmittel oder Bestandteile der Zubereitungen, die in dieser Weise, oder Calciumionen bindend wirken, nicht oder nur in geringer Menge in den Zubereitungen enthalten. Durch zusätzliche Dosierung von Ca-Ionen oder höhere Anteile der Calciumsalze in der erfindungsgemäßen Kombination können aber diese Einwirkungen gegebenenfalls auch, wie dem Fachmann bekannt, ausgeglichen werden. Vorzugsweise liegt der Anteil von Calciumbindemitteln, falls diese gewünscht sind, z.B. unter 0,001 Gew.-% bis 0,01 Gew.-%, insbesondere aber unter 0,1 Gew.-%, jeweils bezogen auf das Gewicht der Zubereitungen.

Das Gewichtsverhältnis der Wirkstoffe Oryzanol/Calciumsalze zueinander in den Kombinationen kann in einem breiten Bereich variiert werden und kann beispielsweise 100 : 1 bis 1 : 100, vorzugsweise 10 : 1 bis 1 : 10 und insbesondere aber auch 1:1 betragen.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure Phosphat-Puffergemischen. Vorzugsweise liegt der pH-Wert unter 10, z.B. im Bereich von 3 bis 9.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.

Als Gamma-Oryzanol wird in den Beispielen das Handelsprodukt Gamma-Oryzanol der Firma Jan Dekker, DE, verwendet.

Als PVPNA-Copolymerisat wird in den Beispielen Luviskol VA 64 W (BASF) verwendet.

Die Mengenangaben in den Beispielen sind Gew.-%.

### Beispiele 1 und 2

### Schaumfestiger

| | 1 | 2 |
|---|---|---|
| PVPNA Copolymer | 8,0 | 8,0 |
| Hydroxyethyl Cetyldimonium Phosphate | 0,1 | 0,1 |
| Gamma-Oryzanol | 0,1 | 0,05 |
| Calziumpanthotenat | 0,07 | 0,1 |
| Parfüm, Lösungsvermittler, | | |
| Pflegestoffe | q.s. | q.s. |
| Ethanol abs. | 10,0 | 10,0 |
| Propan/Butan | 10,0 | 10,0 |
| Wasser, VES (vollentsalzt) | ad 100,0 | ad 100,0 |

### Beispiele 3 und 4

| | 3 | 4 |
|---|---|---|
| PVP/VA Copolymer | 5,0 | 5,0 |
| Polyquaternium-16 | 2.0 | 2.0 |
| Hydroxyethyl Cetyldimonium Phosphate | 0,1 | 0,1 |
| Gamma-Oryzanol | 0,1 | 0,05 |
| Calziumpanthotenat | 0,07 | 0,1 |
| Parfüm, Lösungsvermittler, | | |
| Pflegestoffe | q.s. | q.s. |
| Ethanol abs. | 10,0 | 10,0 |
| Propan/Butan | 10,0 | 10,0 |
| Wasser, VES | ad 100,0 | ad 100,0 |

### Beispiele 5 und 6

### Stylinggele

| | 5 | 6 |
|---|---|---|
| PVP/VA Copolymer | 5,0 | 5,0 |
| Ceteareth-25 | 0,1 | 0,1 |
| Carbomer | 0,8 | 0,8 |
| Gamma-Oryzanol | 0,1 | 0,05 |
| Calziumpanthotenat | 0,07 | 0,1 |
| Parfüm, Lösungsvermittler, Pflegestoffe | | |
| Neutralisationsmittel/pH-Einstellung | q.s. | q.s. |
| Ethanol abs. | 10,0 | 10,0 |
| Wasser, VES | ad 100,0 | ad 100,0 |

## Patentansprüche

1. Stylingmittel auf Wasserbasis, **dadurch gekennzeichnet, daß** sie
a) Filmbildner,
b) Gamma-Oryzanol und
c) ein Calciumsalz oder mehrere Calciumsalze, ausgewählt aus der Gruppe, gebildet von Calciumpanthotenat, Calciumchlorid und Calciumlactat, enthalten.

2. Zubereitungen nach Anspruch 1, **dadurch gekennzeichnet, daß** zusätzlich weitere Tenside und/oder kosmetische oder dermatologische Hilfs-, Zusatz- und/oder Wirkstoffe enthalten sind.

3. Zubereitungen nach Anspruch 1, **dadurch gekennzeichnet, daß** der Gehalt an Oryzanol 0,001 bis 5,0 Ges.-%, bezogen auf das Gesamtgewicht der Zubereitungen, beträgt

4. Zubereitungen nach Anspruch 1, **dadurch gekennzeichnet, daß** der Gehalt an einem oder mehreren Calciumsalzen 0,01 bis 25 Gew.-%, bevorzugt 0,02 bis 5 Gew.-%, jeweils bezogen auf das Gesamtgewicht der Zubereitungen, beträgt

5. Zubereitungen nach einem der vorhergehenden Ansprüche, **dadurch gekennzeichnet, daß** die Gesamtmenge der Filmbildnersubstanzen zwischen 0,5 und 20 Ges.-%, bezogen auf das Gesamtgewicht der Zubereitung beträgt

6. Zubereitungen nach einem der vorhergehenden Ansprüche, **dadurch gekennzeichnet, daß** die Filmbildner gewählt werden aus der Gruppe der wasserlöslichen oder wasserdispergierbaren Polyurethane, Polyharnstoffe, Silikonharze und/oder Polyester sowie der nichtionischen, anionischen, amphoteren und/oder kationischen Polymere.

7. Zubereitungen nach einem der vorhergehenden Ansprüche, **dadurch gekennzeichnet, daß** der pH-Wert der Zubereitungen unter 10 liegt.

8. Zubereitungen nach einem der vorhergehenden Ansprüche, **dadurch gekennzeichnet, daß** der Wassergehalt der Zubereitungen 50 bis 95 Gew.-% beträgt

## Claims

1. Water-based styling compositions **characterized in that** they comprise
a) film formers,
b) gamma-oryzanol and
c) one or more calcium salts chosen from the group formed by calcium panthotenate, calcium chloride and calcium lactate.

2. Preparations according to Claim 1, **characterized in that** further surfactants and/or cosmetic or dermatological auxiliaries, additives and/or active ingredients are additionally present.

3. Preparations according to Claim 1, **characterized in that** the content of oryzanol is 0.001 to 5.0% by weight, based on the total weight of the preparations.

4. Preparations according to Claim 1, **characterized in that** the content of one or more calcium salts is 0.01 to 25% by weight, preferably 0.02 to 5% by weight, in each case based on the total weight of the preparations.

5. Preparations according to one of the preceding claims, **characterized in that** the total amount of film former substances is between 0.5 and 20% by weight, based on the total weight of the preparation.

6. Preparations according to one of the preceding claims, **characterized in that** the film formers are chosen from the group of water-soluble or water-dispersible polyurethanes, polyureas, silicone resins and/or polyesters, and nonionic, anionic, amphoteric and/or cationic polymers.

7. Preparations according to one of the preceding claims, **characterized in that** the pH of the preparations is below 10.

8. Preparations according to one of the preceding claims, **characterized in that** the water content of the preparations is 50 to 95% by weight.

## Revendications

1. Préparations de coiffage à base aqueuse, **caractérisées en ce qu'**elles contiennent
a) un agent filmogène,
b) du gamma-oryzanol et
c) un sel de calcium ou plusieurs sels de calcium, choisi(s) dans le groupe constitué par le pantothénate de calcium, le chlorure de calcium et le lactate de calcium.

2. Préparations selon la revendication 1, **caractérisées en ce qu'**en outre d'autres tensioactifs et/ou des adjuvants, additifs et/ou principes actifs cosmétiques ou dermatologiques sont contenus.

3. Préparations selon la revendication 1, **caractérisées en ce que** la teneur en oryzanol va de 0,001 à 5,0 % en poids, par rapport au poids total des préparations.

4. Préparations selon la revendication 1, **caractérisées en ce que** la teneur en un ou plusieurs sels de calcium va de 0,01 à 25 % en poids, de préférence de 0,02 à 5 % en poids, chaque fois par rapport au poids total des préparations.

5. Préparations selon l'une quelconque des revendications précédentes, **caractérisées en ce que** la quantité totale des substances filmogènes est comprise entre 0,5 et 20 % en poids, par rapport au poids total de la préparation.

6. Préparations selon l'une quelconque des revendications précédentes, **caractérisées en ce que** les agents filmogènes sont choisis dans le groupe des polyuréthannes, polyurées, résines silicone et/ou polyesters, solubles dans l'eau ou dispersables dans l'eau, ainsi que des polymères non ioniques, anioniques, amphotères et/ou cationiques.

7. Préparations selon l'une quelconque des revendications précédentes, **caractérisées en ce que** le pH des préparations est inférieur à 10.

8. Préparations selon l'une quelconque des revendications précédentes, **caractérisées en ce que** la teneur en eau des préparations va de 50 à 95 % en poids.
